Europäisches Patentamt

European Patent Office

Office européen des brevets

(19)

(11) Numéro de publication: **0 170 534 B1**

## (12) FASCICULE DE BREVET EUROPEEN

(45) Date de publication de fascicule du brevet:
24.07.91

(51) Int. Cl.5: **B01J 31/28**, C07C 29/15, C07C 1/06

(21) Numéro de dépôt: **85400857.0**

(22) Date de dépôt: **02.05.85**

(54) Procédé d'hydrocondensation du monoxyde de carbone.

(30) Priorité: 04.05.84 FR 8406923
24.05.84 FR 8408124

(43) Date de publication de la demande:
05.02.86 Bulletin 86/06

(45) Mention de la délivrance du brevet:
24.07.91 Bulletin 91/30

(84) Etats contractants désignés:
AT BE CH DE FR GB IT LI LU NL SE

(56) Documents cités:
FR-A- 1 350 644       FR-A- 1 575 779
FR-A- 1 583 177       FR-A- 2 391 978
GB-A- 1 151 005       US-A- 4 195 042
US-A- 4 390 729

JOURNAL OF THE CHEMICAL SOCIETY, Chemical Communication, 1980, The Chemical Society, pages 908-909, Letchworth, Herts, GB; M. BLANCHARD et al.: "Cobalt catalysts for the liquid phase synthesis of light olefins from CO and H2"

(73) Titulaire: **ATOCHEM**
**4 & 8, Cours Michelet La Défense 10**
**F-92800 Puteaux(FR)**

(72) Inventeur: **Simon, Michel Lucien**
**35 rue de Stalingrad**
**F-62440 Harnes(FR)**
Inventeur: **Petit, Françis**
**27 Avenue de Flandres**
**F-59290 Wasquehal(FR)**
Inventeur: **Caze, Claude**
**6, Sentier des Filatiers**
**F-59810 Lesquin(FR)**

(74) Mandataire: **Rochet, Michel et al**
**ATOCHEM Département Propriété Industriel-le La Défense 10 Cédex 42**
**F-92091 Paris La Défense(FR)**

## Description

La présente invention concerne un procédé d'hydrocondensation du monoxyde de carbone.

Il est déjà connu, notamment par M. BLANCHARD, D. VANHOVE, F. PETIT et A. MORTREUX, J.C.S. Chem. Comm., 1980, pages 908-909, de synthétiser une coupe d'oléfines et d'alcanes ayant de 1 à 6 atomes de carbone à partir de monoxyde de carbone et d'hydrogène dans un rapport $CO/H_2$ compris entre 0,5 et 1, par réaction sous pression atmosphérique et à 199° C en présence de butadiène, d'acétylacétonate de cobalt II et de triéthylaluminium, la durée de la réaction étant supérieure ou égale à 1 heure. Le brevet FR-A-1.583.177 décrit un procédé d'hydrogénation d'un polymère au moyen d'un catalyseur comprenant un hydrocarbure insaturé, un composé organique d'un metal choisi parmi nickel, cobalt et fer, et un agent réducteur composé métal.

Un but de la présente invention consiste en la synthèse d'hydrocarbures et de composés oxygénés aliphatiques à partir de monoxyde de carbone et d'hydrogène en présence d'un catalyseur avec un taux de conversion et une sélectivité élevés.

Pour atteindre le but précité, la demanderesse propose un procédé d'hydro-condensation du monoxyde de carbone, caracterisé en ce que la réaction est effectuée avec un rapport molaire $CO/H_2$ compris entre 0,2 et 2, sous une pression comprise entre 5 et 400 bars, à une température comprise entre 210° et 400° C, le temps de séjour du mélange réactionnel étant compris entre 0,1 et 120 secondes et en présence d'au moins une espèce catalytique obtenue par mise en contact de:

(a) au moins un complexe de formule $ML_n$ dans laquelle M est un métal choisi parmi le fer et le cobalt et le rhodium, n est le nombre de ligands complexant le métal M et L est un ligand ionique ou covalent,

(b) au moins un activateur de formule $M'R_m$ dans laquelle M' est un métal choisi parmi aluminium, lithium, sodium, magnésium et zinc, m est la valence du métal M' et R est choisi parmi l'atome d'hydrogène et les radicaux hydrocarbonés, le rapport molaire (b)/(a) étant compris entre 0,3 et 20, et

(c) au moins un composé choisi parmi les diènes conjugués, les phosphines, les amines, les arsines et les phosphites, le rapport molaire (c)/(a) étant compris entre 1 et 20.

Ainsi le procédé selon l'invention comprend la mise en contact d'au moins trois composants dans des proportions bien définies. Dans le cadre de cette définition générale, la nature de chaque composant peut être précisée comme suit. Le composant (a) est un complexe ionique ou covalent du cobalt dans lequel les ligands L peuvent être choisis notamment parmi les cétones (en particulier l'acétylacétone) et les anions chlorate, nitrate, carboxylates et halogènes. Le cobalt peut bien entendu être entouré de ligands identiques ou différents. Le composant (b) est un activateur du cobalt, jouant le plus souvent mais non exclusivement le rôle de réducteur et comprenant des liaisons métal-carbone ou métal-hydrogène ; son rapport molaire au composant (a) peut être plus particulièrement compris entre 0,5 et 5. Les radicaux hydrocarbonés liés au métal M' dans le composant (b) peuvent être choisis notamment parmi les radicaux alkyles, cycloalkyles, aryles, arylalkyles et alkylaryles ayant de 1 à 12 atomes de carbone. Des exemples de composants (b) sont en particulier les alkyl-lithiens RLi, les dialkylzincs $R_2Zn$, les trialkylaluminiums $AlR_3$, l'hydrure double d'aluminium et lithium $LiAlH_4$ et le borohydrure de sodium $NaBH_4$. Les radicaux hydrocarbonés liés au métal M' peuvent bien entendu être identiques ou différents. Le composant (c) peut être en particulier le norbornadiène ou le butadiène - 1,3 et son rapport molaire au composant (a) peut être plus particulièrement compris entre 3 et 10.

L'espèce catalytique peut être déposée sur un support, par exemple un polymère organique poreux. Des exemples de polymères adaptés au dépôt d'un tel catalyseur sont notamment des copolymères styrène-divinylbenzène et les copolymères 4-vinylpyridine/divinylbenzène rendus poreux par l'addition, pendant leur synthèse, d'au moins un agent porogène adapté aux comonomères présents, tel que par exemple l'heptane ou l'acide éthyl-2 hexanoïque. De tels copolymères ont en effet une structure poreuse dont la surface spécifique est inférieure ou égale à 250 m²/g et le volume poreux est inférieur ou égal à 2 cm³/g. Dans un tel cas, il est nécessaire que la température d'utilisation du catalyseur soit inférieure à la température de décomposition du support polymère, celle-ci étant généralement comprise entre 250° et 290° C pour les copolymères cités précédemment. L'espèce catalytique peut également être déposéesur un support minéral tel que l'alumine, la silice, les silice-alumines ou incorporé dans une zéolithe suivant les techniques bien connues de l'homme de l'art. l'espèce catalytique peut comprendre en outre (d) au moins un composé de formule $M''L'_p$ dans laquelle M'' est un métal choisi parmi manganèse, zinc et chrome, p est le nombre de ligands complexant M'' et L'est un ligand ionique ou covalent.

Un procédé de préparation du catalyseur décrit précédemment, consiste en la mise en contact des composants (a), (b) et (c) dans un moins un solvant aromatique essentiellement anhydre et au moins partiellement sous atmosphère de gaz inerte. Par gaz inerte au sens de la présente invention on entend au moins un gaz choisi parmi hydrogène, azote, monoxyde de carbone et gaz rares. La mise en contact des composants peut être effectuée à une température comprise entre 0° et + 60°C et en une ou plusieurs étapes. Lorsque le procédé de préparation est réalisé en une seule étape, il est essentiel que celle-ci soit conduite sous atmosphère de gaz inerte. Lorsque le procédé selon l'invention est réalisé en plusieurs étapes dont l'une est l'étape d'introduction du composant (b), cette étape peut être conduite sous atmosphère d'un gaz réducteur tel que l'hydrogène. Divers solvants aromatiques, tels que notamment benzène ou toluène, peuvent convenir au procédé selon l'invention sous réserve qu'ils soient véritablement anhydres.

Ainsi comme exemple d'un procédé en plusieurs étapes on peut citer un mode de réalisation consistant, dans une première étape, à solubiliser au moins un composant (a) dans le benzène anhydre, dans une seconde étape à introduire le composant (b) sous atmosphère d'hydrogène et à le laisser réagir avec le composant (a) pendant quelques minutes et dans une troisième étape à introduire le composant (c) solubilisé dans le benzène anhydre.

Le procédé selon l'invention peut être effectué en présence d'au moins un solvant ayant un point d'ébullition supérieur ou égal à la température de réaction. De préférence un tel solvant n'est ni hydrogénable ni hydrogénolysable dans les conditions de ladite réaction et peut être choisi par exemple parmi le biphényle, l'ortho-terphényle, la décaline, la tétraline et le tétraglyme. Lorsque le catalyseur est isolé à l'état solide ou déposé sur un support, la réaction peut aussi être effectuée selon les principes de la catalyse hétérogène en phase gazeuse en mettant en oeuvre soit une technologie en lit fixe, soit un technologie en lit fluidisé soit une technologie en lit entraîné bien connues de l'homme de l'art.

Le procédé de synthèse selon l'invention permet d'obtenir, à partir de monoxyde de carbone et d'hydrogène, des mélanges d'alcanes d'oléfines et de composés oxygénés ayant de 1 à 8 atomes de carbone.

L'essentiel des composés oxygénés est constitué d'alcools normaux ayant de 1 à 4 atomes de carbone, tels que méthanol, éthanol, n-propanol et n-butanol, les autres composés oxygénés éventuellement présents étant des aldéhydes, des cétones, des alcools ramifiés et des esters. La constitution de ces mélanges dépend bien entendu des conditions de synthèse, en particulier la pression, la température, le temps de séjour du mélange réactionnel et la formule chimique du catalyseur.

Les exemples ci-après sont donnés à titre illustratif et non limitatif de la présente invention.

Exemple 1

Dans un tube de Schlenk on introduit, sous agitation et sous atmosphère d'azote, 13,1 g d'acétylacétonate de cobalt que l'on solubilise dans 300 ml de benzène anhydre. On introduit ensuite 6 ml de triéthylaluminium sous atmosphère d'hydrogène. Après un temps de 5 minutes on ajoute 14 g de butadiène -1,3 solubilisé dans 100 ml de benzène anhydre.

Exemple 2 à 5

A la solution de catalyseur dans le benzène préparée conformément à l'exemple 1 on ajoute 300 g d'ortho-terphényle puis on introduit la solution dans un réacteur autoclave de volume 1 litre et la distillation discontinue du benzène est conduite en présence du gaz de synthèse constitué d'un mélange équimolaire de monoxyde de carbone et d'hydrogène. L'hydrocondensation du monoxyde de carbone dans un rapport $CO/H_2$ égal à 1 est ainsi effectuée sous une pression de 30 bars, à une température de 260°C et avec un temps de séjour t du mélange réactionnel (exprimé en secondes) indiqué dans le tableau ci-après. En sortie de réacteur, après détente des gaz, une série de chromatographes en phase gazeuse permet d'analyser et d'identifier les différents produits de synthèse. On trouvera dans le tableau I ci-après :
- le taux de conversion T.C. égal au nombre de moles de CO consommées sur le nombre de moles de CO introduites,
- la sélectivité en hydrocarbures H.C., égale au rapport du nombre de moles de CO transformées en hydrocarbures au nombre de moles de CO transformées, exprimée en pourcent (la conversion de CO en $CO_2$ n'est pas prise en compte)
- la sélectivité en produits oxygénés P.O., égale au rapport du nombre de moles de CO transformées en produits oxygénés au nombre de moles de CO transformées (la conversion de CO en $CO_2$ n'est pas prise en compte)
- les caractéristiques des coupes d'hydrocarbures et de composés oxygénés exprimée en pourcentage molaire.

Exemple 6

3

L'hydrocondensation du monoxyde de carbone dans un rapport CO/H$_2$ égal à 0,5 est effectuée, en utilisant la même solution de catalyseur qu'aux exemples précédents, sous une pression de 100 bars et à une température de 250°C. En sortie de réacteur, les différents produits de synthèse sont analysés comme précédemment et les résultats figurent dans le tableau I ci-après.

Exemple 7

Dans un tube de Schlenk on introduit, sous agitation et sous atmosphère d'azote, 7,8 g d'acétylacétonate de cobalt et 7,7 g d'acétylacétonate de manganèse que l'on solubilise dans 350 ml de benzène anhydre. On introduit ensuite 60 ml d'une solution de diéthylzinc dans l'heptane à 1,25 mole/litre. Puis on ajoute 29,2 g de butadiène-1,3 solubilisé dans 50 ml de benzène anhydre.

Exemple 8

A la solution de catalyseur préparée conformément à l'exemple 7 on ajoute 300 g d'ortho-terphényle puis on introduit la solution dans un réacteur autoclave de volume 1 litre et la distillation discontinue du benzène est conduite en présence du gaz de synthèse constitué d'un mélange équimolaire de monoxyde de carbone et d'hydrogène. L'hydrocondensation du monoxyde de carbone dans un rapport CO/H$_2$ égal à 0,5 est ainsi effectuée sous une pression de 41 bars et à une température de 280°C. En sortie de réacteur, les différents produits de synthèse sont analysés comme précédemment ; les résultats obtenus figurent dans le tableau I ci-après (le complément à 100 % de la coupe de produits oxygénés est constitué par 8,2 % d'acétone, 5,1 % d'acétate de méthyle, 1,1 % de méthyléthylcétone et 1,1 % d'acétate d'éthyle).

Exemple 9

Dans un ballon de Schlenk on introduit, sous agitation et sous atmosphère d'azote, 18 g d'acétylacétonate de cobalt (II) que l'on solubilise dans 150 ml de benzène anhydre. On introduit ensuite 65 ml d'une solution de diéthylzinc dans l'heptane à 1,25 mole/litre, ceci sous atmosphère d'hydrogène. Puis on ajoute 30 g de butadiène-1,3 solubilisé dans 40 ml de benzène anhydre. Après réaction, on réduit le volume de la solution catalytique à 120 ml par distillation sous vide du benzène. On ajoute ensuite 230 g de billes d'alumine SCS 79 (RHONE-PROGIL). Après absorption de la solution catalytique, on sèche les billes imprégnées sous vide jusqu'à élimination du benzène.

Exemple 10

On introduit 90 g de billes préparées conformément à l'exemple 9 dans un réacteur tubulaire, ceci sous atmosphère inerte. Après une période d'activation sous hydrogène de 7 heures de 250°C à 360°C sous un débit de 5 litres/minute, l'hydrocondensation du monoxyde de carbone dans un rapport CO/H$_2$ égal à 0,5 est effectuée sous une pression de 10 bars, à une température de 370°C et avec un temps de séjour t mentionné dans le tableau 1. En sortie de réacteur, après détente des gaz, les différents produits obtenus sont analysés en chromatographie phase gazeuse.

Exemple 11

Dans un tube de Schlenk on introduit, sous agitation et sous atmosphère d'azote, 18 g d'acétylacétonate de fer que l'on solubilise dans

TABLEAU I

| Exemple | 2 | 3 | 4 | 5 | 6 | 8 | 10 |
|---|---|---|---|---|---|---|---|
| t | 6 | 18 | 59 | 112 | 20 | 16 | 12 |
| T.C. (%) | 4,0 | 6,6 | 17,2 | 23,5 | 28 | 5,5 | 57,8 |
| H.C. | 91,1 | 87,7 | 90,7 | 71,1 | 89,5 | 74,5 | 99,9 |
| P.O. | 8,9 | 12,3 | 9,3 | 28,9 | 10,5 | 25,5 | 0,1 |
| méthane | 69,1 | 68,8 | 68,2 | 82,1 | 68,1 | 68,8 | 67,2 |
| éthane | 8,4 | 9,4 | 9,7 | 9,8 | 9,7 | 13,9 | 10,5 |
| propane | 3,4 | 4,0 | 6,1 | 3,4 | 9,4 | 5,5 | 4,5 |
| butane | 2,0 | 2,1 | 2,8 | 1,0 | 5,7 | 2,2 | 2,1 |
| alcanes $C_5$-$C_8$ | 2,0 | 2,2 | 2,8 | 0,7 | 2,0 | 1,9 | 2,3 |
| éthylène | 2,1 | 1,6 | 0,8 | 0,3 | 0,1 | 0,8 | 1,3 |
| propène | 8,0 | 7,4 | 5,9 | 1,8 | 2,6 | 4,8 | 7,2 |
| butène-1 | 2,5 | 2,1 | 1,7 | 0,3 | 0,7 | 1,3 | 0,8 |
| butène-2 | 0,5 | 0,7 | 0,6 | 0,3 | 0,6 | 0,3 | 1,8 |
| alcènes $C_5$-$C_8$ | 2,0 | 1,7 | 1,4 | 0,2 | 1,1 | 0,5 | 2,3 |
| méthanol | 54,0 | 49,7 | 46,3 | 62,9 | 56,9 | 43,0 | 23,5 |
| éthanol | 32,2 | 32,8 | 36,7 | 27,8 | 24,2 | 27,9 | 42,7 |
| n-propanol | 10,3 | 13,3 | 12,0 | 6,3 | 11,6 | 7,1 | -- |
| n-butanol | 2,2 | 2,1 | 2,8 | 1,0 | 3,7 | 3,1 | -- |
| acétaldéhyde | 0,6 | 0,7 | 0,6 | 0,7 | 1,0 | 0,8 | -- |
| alcools $C_3$-$C_4$ ramifiés | 0,7 | 1,4 | 1,6 | 1,3 | 2,6 | 2,5 | 33,8 |

EP 0 170 534 B1

Exemples 12 à 16

150 ml de benzène anhydre. On introduit ensuite 67 ml d'une solution benzénique de triéthylaluminium à 1 mole/1 sous atmosphère d'hydrogène. Après un temps de 10 minutes on ajoute 15 g de butadiène-1,3 solubilisé dans 100 ml de benzène anhydre.

A la solution benzénique de catalyseur préparée conformément à l'exemple 11 on ajoute 300 g d'ortho-terphényle puis on introduit la solution dans un réacteur autoclave de volume 1 litre et la distillation discontinue du benzène est conduite en présence du gaz de synthèse constitué d'un mélange équimolaire de monoxyde de carbone et d'hydrogène. L'hydrocondensation du monoxyde de carbone dans un rapport $CO/H_2$ égal à 1,1 (à l'exception de l'exemple 16 : rapport égal à 0,5) est ainsi effectuée sous une pression P (exprimée en bars), avec un temps de séjour t du mélange réactionnel (exprimé en secondes) et à une température T (exprimée en degrés Celsius). En sortie de réacteur, après détente des gaz, une série de chromatographes en phase gazeuse permet d'analyser et d'identifier les différents produits de synthèse. On trouvera dans le tableau II ci-après :

- le taux de conversion T.C. égal au nombre de moles de CO consommées sur le nombre de moles de CO introduites,
- la sélectivité en hydrocarbures H.C., égale au rapport du nombre de moles de CO transformées en hydrocarbures au nombre de moles de CO transformées, exprimée en pourcent (la conversion de CO en $CO_2$ n'est pas prise en compte)
- la sélectivité en produits oxygénés P.O., égale au rapport du nombre de moles de CO transformées en produits oxygénés au nombre de moles de CO transformées (la conversion de CO en $CO_2$ n'est pas prise en compte)
- la caractéristique des coupes d'hydrocarbures exprimées en pourcentage molaire
- la caractéristique des coupes de composés oxygénés exprimée en pourcentage molaire.

Pour la compréhension des résultats des exemples 13 et 16, on notera que le complément à 100 % de la coupe de produits oxygénés est constitué par du n-butanol (respectivement 3,9 % et 1,8 %).

Exemple 17

Dans un tube de Schlenk on introduit sous agitation et sous atmosphère d'azote 3,1 g d'acétylacétonate de fer que l'on solubilise dans 70 ml de benzène anhydre. On introduit ensuite 10 g d'un support polymère organique poreux puis 1 ml de triéthylaluminium sous atmosphère d'hydrogène. Après un temps de 5 minutes on ajoute 2 g de butadiène-1,3 solubilisé dans 10 ml de benzène anhydre.

Le support utilisé est un copolymère obtenu par copolymérisation de 60 parties en poids de divinylbenzène pur à 50 % et de 40 parties en poids de 4-vinylpyridine, en suspension dans l'eau en présence de 25 parties en poids d'heptane comme agent porogène et de 1 partie en poids de peroxyde de benzoyle comme initiateur de radicaux libres, la réaction étant effectuée pendant 8 heures à une température de 88° C et suivie de l'extraction de l'agent porogène par le méthanol.

Exemples 18 à 20

A la solution de catalyseur préparée conformément à l'exemple 17, on ajoute 30 g d'orthoterphényle, puis on introduit l'ensemble dans un réacteur autoclave de volume 0,1 litre et la distillation discontinue du benzène est conduite en présence du gaz de synthèse constitué d'un mélange équimolaire de monoxyde de carbone et d'hydrogène.

L'hydrocondensation du monoxyde de carbone est effectuée dans un rapport CO/H2 égal à 1 et les produits de synthèse sont analysés en sortie de réacteur comme indiqué précédemment. Les résultats figurent dans le tableau II ci-après.

Exemple 21

Le catalyseur préparé conformément à l'exemple 17 est utilisé, après distillation continue du benzène, pour l'hydrocondensation du monoxyde de carbone dans un réacteur tubulaire à lit fixe selon le principe de la catalyse hétérogène en phase gazeuse. Le rapport CO/H2 est égal à 0,9. Les produits de synthèse sont analysés en sortie de réacteur comme indiqué précédemment. Les résultats figurent dans le tableau II ci-après.

Exemple 22

Une partie de la solution benzénique de catalyseur préparée conformément à l'exemple 17, est soumise d'abord à une distillation totale du benzène, conduite en présence du gaz de synthèse constitué d'un mélange équimolaire de monoxyde de carbone et d'hydrogène, puis à une évaporation sous vide à 200° C afin d'éliminer tous les produits organiques restants. Le support poreux imprégné de catalyseur est alors utilisé pour l'hydrocondensation du monoxyde de carbone dans un réacteur tubulaire à lit fixe selon le principe de la catalyse hétérogène en phase gazeuse. Le rapport $CO/H_2$ est égal à 0,9. Les produits de synthèse sont analysés en sortie de réacteur comme indiqué précédemment. Les résultats figurent dans le tableau II ci-après.

TABLEAU II

| Exemple | 12 | 13 | 14 | 15 | 16 | 18 | 19 | 20 | 21 | 22 |
|---|---|---|---|---|---|---|---|---|---|---|
| P | 20 | 51 | 50 | 47 | 80 | 30 | 10 | 10 | 10 | 15 |
| t | 66 | 146 | 40 | 10 | 17 | 28 | 14 | 49 | 38 | 3,6 |
| T | 240 | 200 | 240 | 240 | 270 | 230 | 260 | 260 | 260 | 260 |
| T.C.(%) | 1,9 | 2,2 | 2,7 | 1,0 | 8,3 | 0,7 | 1,7 | 6,4 | 1,7 | 1,0 |
| H.C. | 83,0 | 97,4 | 89,7 | 84,0 | 82,2 | 75,5 | 94,4 | 96,4 | 89,4 | 85,2 |
| P.O. | 17,0 | 2,6 | 10,3 | 16,0 | 17,8 | 24,5 | 5,6 | 3,6 | 10,6 | 14,8 |
| méthane | 52,0 | 50,7 | 49,2 | 49,2 | 60,9 | 39,8 | 44,8 | 46,1 | 50,7 | 50,8 |
| éthane | 11,0 | 8,5 | 8,8 | 7,3 | 16,3 | 5,8 | 5,8 | 14,4 | 2,9 | 5,7 |
| propane | 3,5 | 6,6 | 4,6 | 3,3 | 5,7 | 3,6 | 1,6 | 2,9 | 1,2 | 1,5 |
| butane | 2,6 | 3,4 | 3,3 | 2,0 | 2,5 | 2,1 | 0,8 | 1,0 | 0,6 | 0,6 |
| alcanes $C_5$-$C_8$ | 2,4 | 1,9 | 2,0 | 2,6 | 1,6 | 1,7 | 0,7 | 0,8 | 0,4 | 0,9 |
| éthylène | 8,7 | 11,0 | 11,0 | 11,3 | 2,3 | 16,9 | 20,3 | 12,4 | 21,4 | 20,5 |
| propène | 12,2 | 11,4 | 12,3 | 13,3 | 6,8 | 16,8 | 16,0 | 14,9 | 14,1 | 12,9 |
| butène-1 | 4,4 | 4,0 | 5,0 | 5,3 | 1,8 | 7,7 | 5,3 | 3,2 | 5,1 | 3,2 |
| butène-2 | 0,9 | 0,6 | 0,6 | 1,0 | 0,9 | 0,8 | 0,8 | 2,2 | 0,6 | 1,3 |
| alcènes $C_5$-$C_8$ | 2,3 | 1,9 | 3,2 | 4,7 | 1,2 | 4,8 | 3,9 | 2,1 | 3,0 | 2,6 |
| méthanol | 35,9 | 27,0 | 26,1 | 38,5 | 61,7 | 59,3 | 43,9 | 40,7 | 50,1 | 41,5 |
| éthanol | 45,8 | 25,0 | 37,7 | 36,6 | 26,3 | 33,0 | 42,9 | 48,8 | 45,5 | 52,6 |
| n-propanol | 11,2 | 4,8 | 6,7 | 12,8 | 7,9 | 7,1 | 11,0 | 6,9 | 0,8 | 1,9 |
| acétaldéhyde | 2,2 | 19,4 | 11,0 | 3,2 | -- | 0,5 | 1,3 | 4,2 | 3,7 | 3,9 |
| alcools $C_3$-$C_4$ ramifiés | 4,9 | 19,9 | 18,5 | 8,9 | 2,3 | 0,1 | 0,9 | 0,4 | 0,9 | 0,1 |

7

**Revendications**

1. Procédé d'hydrocondensation du monoxyde de carbone, caractérisé en ce que la réaction est effectuée avec un rapport molaire $CO/H_2$ compris entre 0,2 et 2, sous une pression comprise entre 5 et 400 bars, à une température comprise entre 210° et 400°C, le temps de séjour du mélange réactionnel étant compris entre 0,1 et 120 secondes et en présence d'au moins une espèce catalytique obtenue par mise en contact de :

   a) au moins un complexe de formule $ML_n$ dans laquelle M est un métal choisi parmi le cobalt, le fer et le rhodium, n est le nombre de ligands complexant le métal M et L est un ligand ionique ou covalent,

   (b) au moins un activateur de formule $M'R_m$ dans laquelle M' est un métal choisi parmi aluminium, lithium, sodium, magnésium et zinc, m est la valence du métal M' et R est choisi parmi l'atome d'hydrogène et les radicaux hydrocarbonés, le rapport molaire (b)/(a) étant compris entre 0,3 et 20, et

   (c) au moins un composé choisi parmi les diènes conjugués, les phosphines. les amines, les arsines et les phosphites, le rapport molaire (c)/(a) étant compris entre 1 et 20.

2. Procédé selon la revendication 1, caractérisé en ce que la réaction est éffectuée en présence d'un solvant qui n'est ni hydrogénable ni hydrogénolysable dans les conditions de la réaction.

3. Procédé selon l'une des revendications 1 et 2, caractérisé en ce que le solvant est choisi parmi le biphényle, l'orthoterphényle, la décaline, la tétraline et le tétraglyme.

4. Procédé selon l'une des revendications 1 à 3, caractérisé en ce que l'espèce catalytique est déposée sur un support.

5. Procédé selon la revendication 1, caractérisé en ce que le ligand L est choisi parmi les cétones et les anions chlorate, nitrate, carboxylates et halogènes.

6. Procédé selon la revendication 4, caractérisé en ce que le support est un polymère organique poreux.

7. Procédé selon la revendication 6, caractérisé en ce que le support est choisi parmi les copolymères styrène-divinylbenzène poreux et les copolymères 4-vinylpyridine/divinylbenzène poreux.

8. Procédé selon l'une des revendications 6 et 7, caractérisé en ce que le support polymère poreux a une surface spécifique inférieure ou égale à 250 m²/g.

9. Procédé selon l'une des revendications 1 à 8, caractérisé en ce que le catalyseur comprend en outre (d) au moins un composé de formule $M''L'_p$ dans laquelle M'' est un métal choisi parmi manganèse, zinc et chrome, p est le nombre de ligands complexant M'' et L' est un ligand ionique ou covalent.

10. Procédé selon revendication 4, caractérisé en ce que le support est choisi parmi l'alumine, la silice, les silice-alumines et les zéolithes.

**Claims**

1. Carbon monoxide hydrocondensation process characterised in that the reaction is performed with a $CO/H_2$ molar ratio of between 0.2 and 2, at a pressure of between 5 and 400 bars, at a temperature of between 210° and 400°C, the residence time of the reaction mixture being between 0.1 and 120 seconds, and in the presence of at least one catalytic species obtained by bringing into contact:

   a) at least one complex of formula $ML_n$ in which M is a metal chosen from cobalt, iron and rhodium, n is the number of ligands complexing the metal M, and L is an ionic or covalent ligand,

   b) at least one activator of formula $M'R_m$ in which M' is a metal chosen from aluminium, lithium, sodium, magnesium and zinc, m is the valency of the metal M', and R is chosen from the hydrogen atom and hydrocarbon radicals, the molar ratio (b)/(a) being between 0.3 and 20, and

   c) at least one compound chosen from conjugated dienes, phosphines, amines, arsines and phosphites, the molar ratio (c)/(a) being between 1 and 20.

2. Process according to Claim 1, characterised in that the reaction is performed in the presence of a solvent which is neither capable of being hydrogenated nor capable of being hydrogenolysed under the reaction conditions.

3. Process according to either of Claims 1 and 2, characterised in that the solvent is chosen from biphenyl, ortho-terphenyl, decalin, tetralin and tetraglyme.

4. Process according to one of Claims 1 to 3, characterised in that the catalytic species is deposited on a support.

5. Process according to Claim 1, characterised in that the ligand L is chosen from ketones and chlorate, nitrate, carboxylate and halogen anions.

6. Process according to Claim 4, characterised in that the support is a porous organic polymer.

7. Process according to Claim 6, characterised in that the support is chosen from porous styrene-divinylbenzene copolymers and porous 4-vinylpyridine/ divinylbenzene copolymers.

8. Process according to either of Claims 6 and 7, characterised in that the porous polymer support has a specific surface area lower than or equal to 250 $m^2/g$.

9. Process according to one of Claims 1 to 8, characterised in that this catalyst additionally comprises (d) at least one compound of formula $M''L'_p$ in which $M''$ is a metal chosen from manganese, zinc and chromium, p is the number of ligands complexing $M''$ and $L'$ is an ionic or covalent ligand.

10. Process according to Claim 4, characterised in that the support is chosen from alumina, silica, silica-aluminas and zeolites.

**Patentansprüche**

1. Verfahren zur Hydrokondensation von Kohlenmonoxid, dadurch gekennzeichnet, daß die Reaktion bei einem Molverhältnis von $CO/H_2$ zwischen 0,2 und 2, einem Druck zwischen 5 und 400 bar, einer Temperatur zwischen 210 und 400° C, mit einer Verweilzeit des Reaktionsgemischs zwischen 0,1 und 120 sec und in Gegenwart wenigstens eines katalytischen Stoffes durchgeführt wird, der durch Inberührungbringen

(a) wenigstens eines Komplexes der Formel $ML_n$, in der M ein aus Kobalt, Eisen und Rhodium ausgewähltes Metall, n die Zahl der das Metall M komplexierenden Liganden und L ein ionischer oder kovalenter Ligand ist,

(b) wenigstens eines Aktivators der Formel $M'R_m$, in der M' ein aus Aluminium, Lithium, Natrium, Magnesium und Zink ausgewähltes Metall, m die Wertigkeit des Metalls M' und R aus dem Wasserstoffatom und Kohlenwasserstoffresten ausgewählt ist, wobei das Molverhältnis (b)/(a) zwischen 0,3 und 20 beträgt, und

(c) wenigstens einer Verbindung, die aus den konjugierten Dienen, Phosphinen, Aminen, Arsinen und Phosphiten ausgewählt ist, wobei das Molhältnis (c)/(a) zwischen 1 und 20 beträgt,

erhältlich ist.

2. Verfahren nach Anspruch 1, dadurch gekennzeichnet, daß die Reaktion in Gegenwart eines Lösungsmittels durchgeführt wird, das unter den Reaktionsbedingungen weder hydrierbar noch hydrogenolysierbar ist.

3. Verfahren nach Anspruch 1 oder 2, dadurch gekennzeichnet, daß das Lösungsmittel aus Biphenyl, o-Terphenyl, Decalin, Tetralin und Tetraglym ausgewählt ist.

4. Verfahren nach einem der Ansprüche 1 bis 3, dadurch gekennzeichnet, daß der katalytische Stoff auf einem Träger abgeschieden ist.

5. Verfahren nach Anspruch 1, dadurch gekennzeichnet, daß der Ligand L aus Ketonen und Chlorat-, Nitrat- und Carboxylatanionen und Halogenen ausgewählt ist.

6. Verfahren nach Anspruch 4, dadurch gekennzeichnet, daß der Träger ein poröses organisches Polymeres ist.

7. Verfahren nach Anspruch 6, dadurch gekennzeichnet, daß der Träger aus porösen Styrol-Divinylbenzol- und porösen 4-Vinylpyridin-Divinylbenzol-Copolymeren ausgewählt ist.

8. Verfahren nach Anspruch 6 oder 7, dadurch gekennzeichnet, daß der poröse polymere Träger eine spezifische Oberfläche von höchstens 250 m$^2$/g hat.

9. Verfahren nach einem der Ansprüche 1 bis 8, dadurch gekennzeichnet, daß der Katalysator außerdem (d) wenigstens eine Verbindung der Formel M''L'$_p$ enthält, in der M'' ein aus Mangan, Zink und Chrom ausgewähltes Metall, p die Zahl der M'' komplexierenden Liganden und 1' ein ionischer oder kovalenter Ligand ist.

10. Verfahren nach Anspruch 4, dadurch gekennzeichnet, daß der Träger aus Aluminiumoxid, Siliciumdioxid, Siliciumdioxid-Aluminiumoxiden und Zeolithen ausgewählt ist.